# EUROPEAN PATENT APPLICATION

(11) **EP 1 550 475 A1**
(43) Date of publication of application: **06.07.2005**
(21) Application number: 03771320.3
(22) Date of filing: 24.07.2003
(51) Int. Cl.: A61M 5/158

(54) **HOLE-FORMING PIN FOR INSERTING INDWELLING NEEDLE AND JIG FOR INSTALLING THE PIN**

(30) Priority: 25.07.2002 JP 2002216545
(71) Applicant: Nipro Corporation, Osaka-shi, Osaka 531-8510 (JP)
(72) Inventor: SHINZATO, Toru, Meito-ku, Nagoya-shi, Aichi 465-0021 (JP); TOMA, Shigeki, c/o Touma Clinic, Nakagami-gun, Okinawa 903-0116 (JP); SANO, Yoshihiko, c/o Nipro Corporation, Osaka-shi, Osaka 531-8510 (JP); MASUDA, Toshiaki, c/o Nipro Corporation, Osaka-shi, Osaka 531-8510 (JP); YAMAGUCHI, Futoshi, c/o Nipro Corporation, Osaka-shi, Osaka 531-8510 (JP); HARADA, Kazuyoshi, c/o Nipro Corporation, Osaka-shi, Osaka 531-8510 (JP); TAKAGI, Nobuo, c/o Nipro Corporation, Osaka-shi, Osaka 531-8510 (JP); TANIMOTO, Masahisa c/o Nipro Corporation, Osaka-shi, Osaka 531-8510 (JP)
(74) Representative: Banzer, Hans-Jörg, Dipl.-Ing.
(86) International application number: PCT/JP2003/009417
(87) International publication number: WO 2004/011063

(57) **Abstract**

A pin for forming a hole for inserting an indwelling needle for minimize a load exerted to a living body, and a jig for installing the hole-forming pin from a skin surface are provided.

The pin for forming a hole for inserting an indwelling needle A for holding the indwelling pin from the skin surface to a blood vessel includes a column-shaped insertion part 1 having a curved surface at the distal end thereof, an insertion stop part2 provided at the proximal end of the insertion part 1, and wings 4 connected to the insertion stop part2 via flexible joint branches 3, and a jig for mounting the hole-forming pin A includes a main body 6, a sliding body7 that is built in the main body 6 for sliding in the longitudinal direction of the main body, supporting means 10 for supporting the indwelling needle insertion hole-forming pin at the distal end(s) of the sliding body 7 and/or the main body 6 of the jig for installing the pin are provided, and pin for forming a hole for inserting an indwelling needle A is installing from the skin surface toward the blood vessel by the sliding movement of the sliding body 7.

## Description

### Technical Field

The present invention relates to a pin for forming an insertion hole from a skin surface of a human body to the wall of a blood vessel or to a position close to the wall for inserting a dwelling needle, and a jig for installing the pin.

### Background Art

In general, a hemodialysis patient needs to be subjected to hemodialysis at a rate of twice to three-time a week, and puncturing of the indwelling needle causes the patient to considerable pain every time.

Therefore, in order to minimize the puncturing pain, the applicant has already proposed a hole-forming pin for inserting indwelling (See Publication of Design Registration No. 1136452, No.1137743, No.1138667, No.1140738). These hole-forming pins each comprises a columnar insertion part having a rounded distal end and an insertion stop part provided at the proximal end of the insertion part, and a holding part (recess) provided on the insertion stop part to which the distal end of an insertion assisting device is inserted to retain the pin.

When the pin for forming a hole for inserting an indwelling needle is inserted from a skin surface of a human body to the wall of a blood vessel or to a position close to the wall and left for several days, a hole (hereinafter referred to as a "buttonhole") is formed as a passage for inserting the indwelling needle. After the puncturing, puncturing pain that is caused on puncturing with a method other than this buttonhole-puncturing method can be significantly alleviated since the indwelling needle can be inserted through this buttonhole.

However, the conventional hole-forming pin for inserting indwelling needle has a spherical shape, conical shape, egg shape, or flat shape with a not so large contact area to the skin as the shape of the insertion stop part form, and fixation is not necessarily perfect in indwelling.

For example, in the worst case such that an excessive load is applied to a body (button hole) with the slightest of opportunities as in the case of a touching something and the like, there may be cases a case that button hole are formed in the direction other than the direction to the blood vessel in a state which the swinged insertion part are fixed, or a case that a button hole cannot be formed at all with the detaching of the hole-forming pin. The conventional insertion assisting device for holding the pin for forming a pin for inserting an indwelling needle described above has problems in operability such that it is difficult to check out the direction of fitting the pin, or that the pin itself cannot be removed easily from the insertion assisting device.

### Disclosure of the Invention

In view of such circumstances, it is an object of the present invention to provide a hole-forming pin which can be inserted easily into a puncture passage immediately after removing a dialysis indwelling needle and which can prevent the button hole from being formed in the directions other than the direction toward the blood vessel or from being disconnected easily against the operator's will so as to minimize a load to a living body. And it is an object of the present invention to provide a jig for installing the hole-forming pin (insertion assisting device) with good operability.
As the result of intense study aiming to solve the above-described problems, the present inventors have found that by providing wings on the pin for forming a hole for inserting an indwelling needle, inserting operation can be performed easily because the operator can hold the wings for inserting the hole-forming pin into the puncture passage. The present inventors have found that by fixing the wings with adhesive tape or the like during indwelling, the direction of formation of the button hole can be prevented from changing. The present inventors have found that by providing elastic joint parts at connecting parts between the wings and the insertion stop part, fine adjustment of the angle of insertion of the insertion part is achieved and hence a load exerted on the living body can be minimized. The present inventors have found that consequently reached the present invention. Also, the present inventors have achieved a jig from which the hole-forming pin can be removed at a good timing simultaneously with fitting by insertion of the hole-forming pin from the skin surface.

In other words, the hole-forming pin for inserting indwelling needle of the present invention is a pin for forming a buttonhole from a skin surface to a blood vessel to indwell an indwelling needle. The hole-forming pin for inserting indwelling needle comprises a column-shaped insertion part having a curved surface at the distal end thereof, an insertion stop part provided at the proximal end of the insertion part, and wings connected to the insertion stop part, wherein the insertion stop part and the wings are connected through a elastic joint.

Therefore, although an insertion assisting device to retain the hole-forming pin for inserting the indwelling needle was necessary and a troublesome operation to set the insertion assisting device in the angle of insertion and bring the hole-forming pin apart from the insertion assisting device was necessary in the prior art, according to, since the operator can hold and insert the wing part with fingertips and fix the same in the inserted state, the hole-forming pin for inserting indwelling needle of the present invention have the effect that indwelling operation can be performed easily.

In the hole-forming pin for inserting indwelling needle of the present invention, the outer diameter of the insertion part is from 0.5 to 3.0 mm and the length of the insertion part is from 3 to 20 mm.

In other words, although the diameter is determined by the size of the puncture passage immediately after removal of the dialysis-indwelling needle, it is preferable that the diameter is from about 1.5 mm to 3 mm in consideration of general needle diameter that is from 1.2 mm to 1.8 mm (from 1.5 to 2.3 mm for plastic needle). In addition, the hole-forming pin for inserting indwelling needle in the present application is applied not only to dialysis, but also to regular tests (blood sampling, etc.) or chemical grouting (fluid infusion, etc.), which carried out continuously or discontinuously. The hole-forming pin for inserting indwelling needle can be used with a diameter of from 0.5 mm to 2.0 mm for the needles for blood examination having the diameter from 0.5 mm to 1.8 mm, or for the needles for fluid infusion having the diameter from 1.0 mm to 1.5 mm as needed. The length of the hole-forming pin for inserting indwelling needle in the present application is preferably from 3 to 20 mm, since the distal end of the pin can reach a wall of the blood vessel or a position close to the blood vessel in placing the hole-forming pin.

In the hole-forming pin for inserting indwelling needle according to the present invention, elastic deformation of a elastic joint is such that the entire wings can be moved in the direction of right angle cross-section which is substantially at a right angle to blood flow direction of the blood vessel to which the column-shaped insertion part is to be inserted with respect to the insertion stop part.

By this, since the insertion part and the wings are flexibly connected via the elastic joint part s, opening and closing of the wings and insertion operation are good because can be easily performed. Therefore operability of the insertion can be easily performed. Since the fixing force of the wings transmitted to the insertion part is relaxed in fixing the pin and the load for the living body can be preferably reduced, it is preferable to connect via the elastic joint parts flexibly.

In the hole-forming pin for inserting indwelling needle of the present invention, elastic deformation at the elastic joint parts allows the column-shaped insertion part to have flexibility in puncturing angle to the blood vessel with respect to the wings. In this case, even when the angle of the puncture passage (button hole) after having removed the indwelling needle and the angle of puncture of the hole-forming pin are not exactly same, the pin can be inserted smoothly along the angle of the button hole by inserting the insertion part therethrough.

In the pin for forming a hole for inserting an indwelling needle according to the present invention, elastic deformation at the elastic joint allows the column-shaped insertion part at the insertion stop part to have angular flexibility substantially in the rotating direction about the axis of the column-shaped part of the column-shaped insertion part with respect to the wings.

In this case as well, even when the puncture passage after having removed the indwelling needle is formed not exactly above in the direction of the blood vessel, but formed slightly obliquely toward the blood vessel, the hole-forming pin can be inserted smoothly and, even the insertion operation can be performed without increasing the load exerted on the living body.

In the hole-forming pin for inserting indwelling needle according to the present application, when the elastic deformation at the elastic joint is set based on the material and the shape of the elastic joint parts so that the balance with the elastic deformation for allowing the column-shaped insertion part to have flexibility in puncturing angle to the blood vessel with respect to the wings and the elastic deformation for allowing the column-shaped insertion part at the insertion stop part to have angular flexibility substantially in the rotating direction about the axis of the column-shaped part of the column-shaped insertion part with respect to the wings are adjusted respectively, even when the angle of the insertion part does not exactly match the puncture passage after having removed the indwelling needle, the insertion part of the hole-forming pin can be inserted smoothly along the button hole as long as both of the aforementioned angles are within the angular error on the order of ±2 to 10° for each direction.

Then, when the pin for forming a hole for inserting an indwelling needle of the present invention is configured in such a manner that the wings are connected to the insertion stop part via flexible joint branches, the hole-forming pin having superior operability with suitably balanced angular flexibility in terms of the puncturing angle with respect to the blood vessel to which the column-shaped insertion part is inserted and of the angle at which the insertion stop part in association with the column-shaped insertion part substantially rotates about the axis of the column-shaped part of the column-shaped insertion part can be obtained, and the load exerted to the living body can be minimized. Among others, in view of practicability, the dimensions of the joint branches are most preferably from 0.1 to 2 mm in diameter of the lateral cross-section, and 0.5 to 10 mm in length.

The term "joint branch" is an appellation of an embodiment of the elastic joint part which connect the insertion stop part and the wings of the pin for forming a hole for installing an indwelling needle, and represents the part extending from the insertion stop part in a branch shape and connecting the insertion stop part and the wings.

The jig for installing used for the pin for forming a hole for inserting an indwelling needle of the present invention is a jig for installing the above-described hole-forming pin from the skin surface toward the blood vessel including supporting means having a main body and a sliding body that is built in the main body for sliding in the longitudinal direction of the main body, the supporting means supporting the hole-forming pin at the distal end(s) of the main body and/or the sliding body. Therefore, it is preferable to use the jig for installing the hole-forming pin for inserting indwelling needle, since no uncertainty of installation such as the case of inserting with fingertips, insertion with smoothness and mounting of the hole-forming pin along the button hole can be achieved with smooth installing hole-forming pin from the skin surface to the blood vessel, which are supported by the support means by moving the slide part built in the main body.

The jig for installing pin according to the present invention comprises two opposite side-plates extending in parallel with the main body, which are formed integrally in the main body. The sliding body can slide forwardly of the main body with a guide that engages the inner walls of the two side plates which constitute the main body and the outer wall which constitutes the sliding body, or comprise the two spring parts bending in the outward direction of the main body and the sliding body, the two spring parts are respectively fixed at the ends thereof to the left and right side walls of the main body and the left and right side walls of the sliding body, wherein the sliding body is inserted slidably into a sliding shaft projecting from the distal end of the main body

The forwardly of the main body of the jig for installing represents the side on which the pin for forming a hole for inserting an indwelling needle is attached and the forming pin is loaded from the skin surface toward the blood vessel.

It is preferable to use the jig for installing the pin of the present invention wherein the sliding body is provided with driving means which allows the sliding body to slide easily along the main body with smooth sliding movement enabled, a finger hook part formed on a part of the sliding body is used as the driving means for the sliding body due to its simple operationality.
Since the easier and stable operability can be preferably obtained by employing a form in which the energizing force of the spring is utilized for the sliding movement caused by pressing the finger stop part, a constant operating force which does not vary depending on the individual difference of the operator like a finger movement, it is preferable to use the jig for installing the pin of the present invention, wherein the driving means for the sliding body is built in the tubular main body formed of left and right side walls, a bottom plate, a top plate and a rear end wall; a coil spring is fitted between the interior of the rear end wall of the main body and the rear end of the sliding body so that the sliding body is energized forward; an upper part of the sliding body is formed with inclined plane facing a forwardly; a inclined plane facing rearwardly corresponding to said inclined plane is formed on the lower part of the finger hook part , and the upper part of the finger hook part projects above the main body through a through-hole formed on the top plate of the main body.

When the jig for installing the pin according to the present application is configured in such a manner that at least one part of the supporting means for supporting the hole-forming pin is moved relatively backward of the distal end of the main body, and the jig has a good operability since the hole-forming pin can be fixed to the skin surface with adhesive tape via the wings by immediate separation of the jig which is not necessary any longer for operation from the hole-forming pin after the hole-forming pin is inserted from the skin surface toward the blood vessel.

When the jig for installing the pin according to the present application is formed with wing holding means for holding the wings of the pin for forming a hole for inserting an indwelling needle at the distal end of the main body and/or the distal end of the sliding body, the distal end of the hole-forming pin is prevented from being unstable in direction because the wings of the hole-forming pin are swung over during mounting operation, and hence the hole-forming pin can be reliably inserted in the predetermined direction, thereby achieving good operability.

### Brief Description of the Drawings

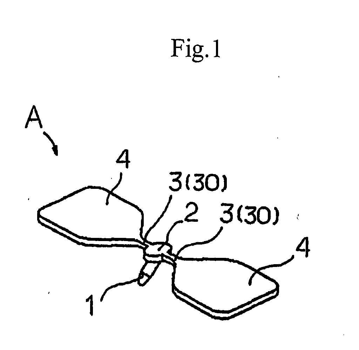

Fig. 1 is a perspective view of one embodiment of the present invention of a pin for forming a hole for inserting an indwelling needle. A pin for forming a hole for inserting an indwelling needle A comprises a column-shaped insertion part 1 having a curved surface at the distal end, an insertion stop part 2 provided on the proximal end of the insertion part 1, and wings 4 connected to the insertion stop part 2 via elastic joint parts 30. In this embodiment, as a best form for implementing the present invention, a case in which the elastic joint parts 30 is formed with a joint branches 3 extending from the insertion stop part, which is a thin and flexible part formed into a branch shape for connecting the insertion stop part and the wings is described.

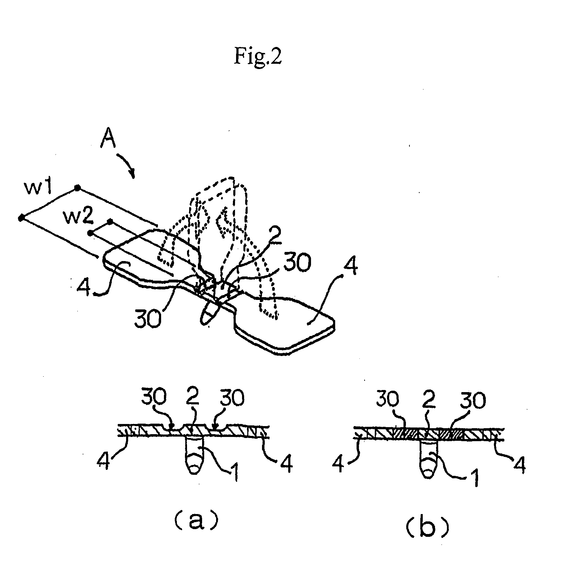

Fig. 2 is a perspective view showing another embodiment of the pin for forming a hole for inserting an indwelling needle according to the present invention, showing an example of the embodiment of the elastic joint parts 30 and the action of the wings 4 whose motion is facilitated thereby.

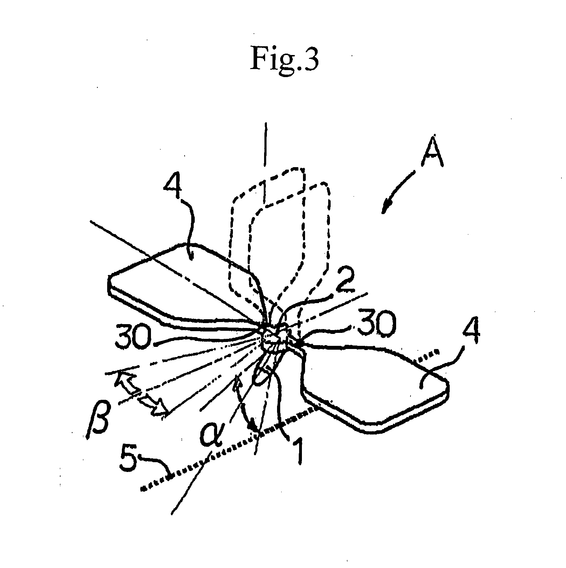

Fig. 3 is a perspective view for explaining followability to the puncture passage when the pin for forming a hole for inserting an indwelling needle of the present invention is applied.

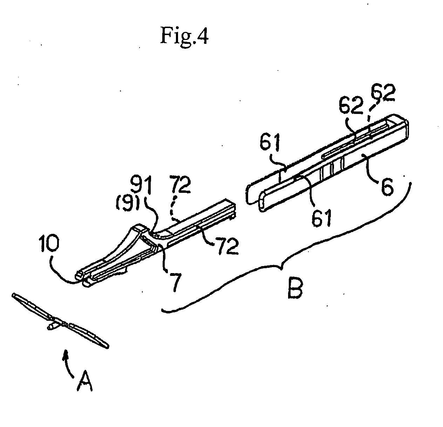

Fig. 4 to Fig. 8 show a jig for installing the pin used for the pin for forming a hole for inserting an indwelling needle of the present invention. A jig B comprises a main body 6, and a sliding body 7 built in the main body 6 for sliding in the longitudinal direction. The sliding body 7 can slid easily by driving means 9, and supporting means 10 for supporting the pin for forming a hole for inserting an indwelling needle A for holding the indwelling needle from the skin surface to the blood vessel is provided on the distal end of the main body 6 and/or the sliding body 7.

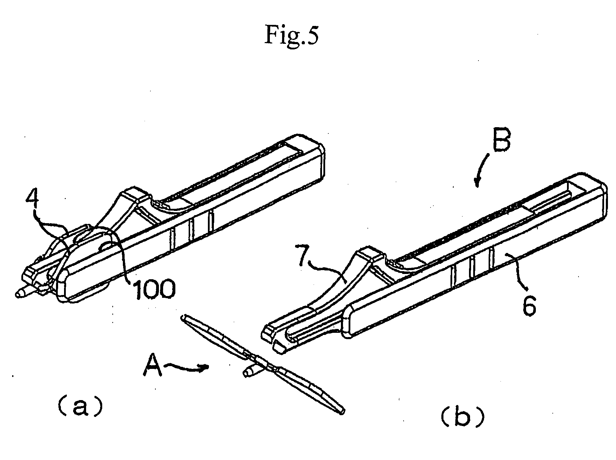

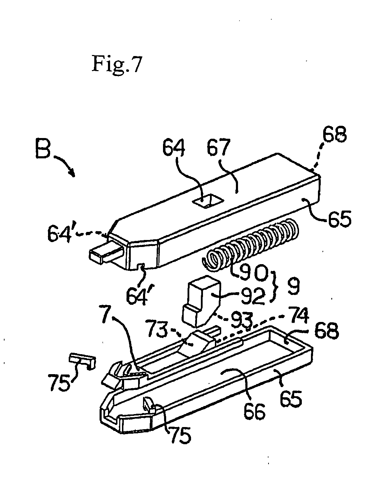

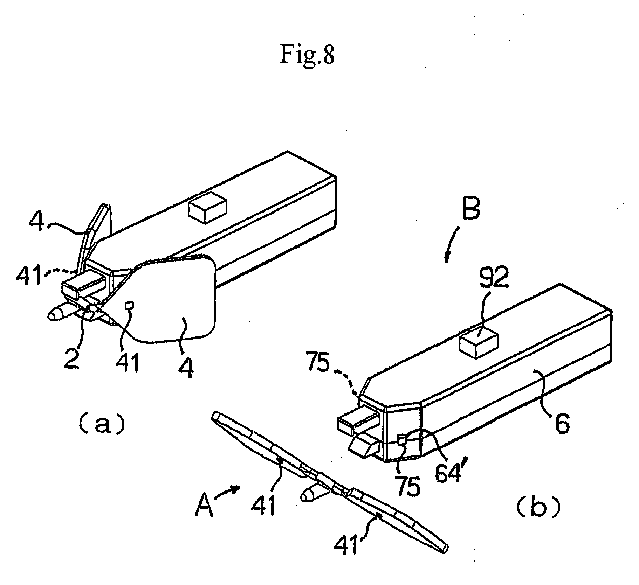

Fig. 4 to Fig. 8 show thee examples for explaining the embodiments respectively in which (a) shows a state in which pin for forming a hole for inserting an indwelling needle is attached to the jig, and (b) shows a state in which the pin for forming a hole for inserting an indwelling needle is separated from the supporting means of the jig (that is, the state after having loaded the hole-forming pin) respectively in Fig 5, Fig 6 and Fig 8. Fig. 4 is an exploded view of Fig. 5, and Fig. 7 is an exploded view of Fig. 8.

### Best Mode for Carrying Out the Invention

Hereinafter referring now to the drawings, the pin for forming a hole for inserting an indwelling needle according to the present invention and the jig thereof will be concretely described based on examples.

### (EXAMPLE 1)

First, the actions in each component of the pin for forming a hole for inserting an indwelling needle A in the present invention will be described.

Fig. 2 shows an example of the embodiment of the pin for forming a hole for inserting an indwelling needle according to the present invention. An insertion stop part 2 is provided at the proximal end of a column-shaped insertion part 1, and wings 4 are connected to both sides of the insertion stop part 2 via elastic joint parts 30. Since the elastic joint parts 30 are provided with higher flexibility than other parts of the hole-forming pin such as the insertion stop part 2 or the wings 4, the wings 4 can be erected to the insertion stop part 2 as shown by a broken line in the drawing by easy bending of the wings formed in a flat state as shown in the drawing.

As described above, the wings 4 standing upright is hold with fingertips, the column-shaped insertion part 1 of the pin for forming a hole for inserting an indwelling needle is inserted into a puncture passage after having removed the indwelling needle, then the wings 4 restored into the flat state again can be fixed by taping from above with adhesive tape or the like.

In addition, in order to provide higher flexibility to the elastic joint parts 30 than the wings 4, the manner such as designing the length of the elastic joint parts 30 (w2 in the drawing) shorter than the width (w1 in the drawing) of the wings 4, providing elastic joint parts 30 formed with partly thinned part as shown in Fig. 2(a), or employing a soft material only for the elastic joint parts 30 as shown in Fig. 2(b) can be used.

In the case of puncturing a needle into a blood vessel, the needlepoint is punctured generally at a suitable angle with respect to the direction of the blood vessel. However, the column-shaped insertion part 1 of the pin for forming a hole for inserting an indwelling needle cannot be necessarily oriented exactly at the angle of puncture passage after having removed the indwelling needle, but in general there arises a certain error in angle.

Then, Fig. 3 shows angles for permitting flexibility required for the followability to the puncture passage given to the column-shaped insertion part 1 of the pin for forming a hole for inserting an indwelling needle

In other words, a shows flexibility of the puncturing angle of longitudinally direction of a blood vessel 5, and b shows flexibility of the angle in the circumferential direction of the axis of the column-shaped part of the column-shaped insertion part of the insertion stop part substantially, respectively.

These flexibilities of angles that are a and b in two directions must be provided based on the material or shape of the elastic joint parts 30 so as to adjust the balance in elastic deformation in the respective directions.

It is preferable that the range of the error in angle is adjusted practically within the range of about ± 2 to 10°, with a determination by the form, material, and dimensions of the elastic joint parts 30 of the pin for forming a hole for inserting an indwelling needle, whereby smooth insertion operation is achieved.

In addition, when the error in angle exceeds the max value of ±10°, the flexibility of the angle becomes too much, and the insertion angle of the column-shaped insertion part 1 becomes unstable, and usability becomes hard by the deterioration of operability. In contrast, when the error in angle is bellow ± 2°, it is too tough for adjustment, and hence it is not suitable for practical use.

The practical selection of the elastic joint parts 30 based on the material or shape is done after making a study of the length W2 as shown in Fig. 2 as an example, thickness, or elasticity depending on the cross-sectional shape or the types of material.

### (EXAMPLE 2)

Referring now to Fig. 1, an example set as a preferred embodiment as described above will be described. In this embodiment, the insertion part 1 is formed by injection molding using a low-density polyethylene.

In the pin for forming a hole for inserting an indwelling needle shown in Fig. 1, a column-shaped insertion part 1 has a shape having a curved surface at the distal end thereof.

With this shape, when forming a button hole by inserting the insertion part 1 to a part punctured by a dialysis indwelling needle or the like, the possibility to give damages to a wall or other parts is eliminated.

In order to form a hole having a size that can accept the indwelling needle with non-sharp distal end, the outer diameter of the insertion part 1 is preferably from 0.5 to 3.0 mm, and more preferably from 1.0 to 2.0 mm, although the application of the indwelling needle described above.

The length of the insertion part 1 is preferably from 3 to 20 mm, and more preferably, from 4.0 to 6.0 mm so that the distal end of the pin can reach a wall of the blood vessel or a position close to the blood vessel in placing the hole-forming pin for the indwelling needle.

Although the insertion part 1 is generally inclined by a suitable angle corresponding to the puncturing angle of the indwelling needle, the present invention is not limited thereto, and the insertion part 1 may be provided perpendicularly to the wings 4. In this embodiment, the entire body is provided by integral molding, and each of the wings 4 is formed to be 10 mm square, and 1 mm in thickness.

The material for forming the insertion part 1 may be synthetic resin such as polyurethane, polypropylene, polycarbonate, ABS resin, polyethylene, polytetrafluoroethylene, and polysulphone or metal such as stainless steel and the like. Materials coated by antithrombotic material or antibacterial material are preferably used, and those materials mixed into the synthetic resin are also preferably used in the case of using the synthetic resin.

The insertion stop part 2 is provided for preventing the insertion part 1 from immersing completely into the skin, and preventing the formation of the entrance of the buttonhole from becoming incomplete.

The shape of the insertion stop part 2 can be employed as spherical shape, plate shape, conical shape, egg-shape or the like. The cross-sectional shape of the insertion stop part2 in the horizontal direction (in the direction in parallel with the surface of the skin which comes into contact therewith) may be any shape as long as there is a part larger than the outer diameter of the insertion part 1 (in the part of maximum diameter). For example, in a circular plate, the maximum diameter is preferably from 2 to 10 mm, and more preferably, from 3.0 to 5.0 mmΦ.

In the example of the embodiment of pin for forming a hole for inserting an indwelling needle according to the present invention shown in Fig. 1, joint branches 3 are employed as parts which are adjusted in balance in elastic deformation in directions within a and b respectively. As described above based on Fig. 3 as one form of the elastic joint parts 30, a shows flexibility of the puncturing angle longitudinally of the blood vessel 5, and b shows angular flexibility of the column-shaped insertion part at the insertion stop part substantially in the rotating direction about the axis of the column-shaped part.

In other words, the joint branches 3 are parts that connect the insertion stop part 2 and the wings 4, and have certain degree of flexibility. Accordingly, when there is difference between the angle of the insertion part 1 to the wings 4 and the angle of the wound (passage) which is a previous stage of a hole to be formed in a state in which the wings 4 described later are fixed to the skin, fine adjustment such as to absorb the difference is effected by twisting of the joint branches 3, so that no load is applied to the living body.

As described above, in order to provide the joint branches 3 to have a suitable flexibility to the insertion stop part 2 or the wings 4, a preferable combination of thickness, length, and material is set. When the insertion part 1 is formed by injection molding of low-density polyethylene and is circular in lateral cross section of the insertion part 1 as in the present embodiment, the diameter is preferably from 0.5 to 3 mmΦ and more preferably from 1 to 2 mmΦ, and the length is preferably from 0.5 to 10 mm, and more preferably from 1 to 5 mm.

The wings 4 work as fixing means for fixing the insertion part 1 tightly to the skin surface. In the case of the pin for forming a hole for inserting an indwelling needle in the prior art, it is necessary to attach a separate auxiliary part to a holding part (recess) formed on the insertion stop part before insertion, and then remove the auxiliary device again after insertion. However, in the case of the pin for forming a hole for inserting an indwelling needle of the present invention, since the operator can hold the wings 4 with fingertips, insert the insertion part 1 and fix without modification, indwelling operation can be performed easily. In this case, it is preferable for medial personnel also to wear medical gloves or the like of plastic film so as to prevent infection and maintaining aseptic condition.

As the shapes of the wings 4, polygonal shape such as triangle or square, the shapes including curved line, such as circular shape, oval shape, or egg shape may also be employed in addition to pentagonal shape shown in Fig. 1 Then, the area of each wing 4 is preferably from 20 to 500 mm², and more preferably from 50 to 200 mm², since the wing is required to have dimensions that make to hold the wings easily in inserting the pin by operator, to fix the wings to the arm or the like, and not to give any load in fixing.

From the viewpoint of minimizing the adhered area of the tape on the skin, it is also possible to cut off one of the two wings 4 shown in the drawing when the insertion part 1 is inserted and the tape may adhere on the remaining wing 4. In this case, since the joint branches 3 are provided with a thinned part having flexibility, one of the two wings 4 can easily be cut off.

In the case of the pin for forming a hole for inserting an indwelling needle according to the present invention, the insertion part 1, the insertion stop part2, the joint branches 3 and the wings 4 are generally formed integrally by injection molding in view of production cost. However, it is also possible to form the insertion part 1 separately, and add the insertion part 1 to the insertion stop part 2 that is formed integrally with the wings 4. The material for the insertion stop part2, the joint branch 3, and the wings 4 in this case may be the same material used for the insertion part 1, and soft material such as the flexible polyvinyl chloride, or rubberlike elastic body can be used the material.

### (Example 1 of the jig for installing the pin)

In Fig. 4 (Fig. 5) showing a first embodiment of a jig for installing the pin B used for the pin for forming a hole for inserting an indwelling needle according to the present invention, the jig B comprises a main body 6 and a sliding body 7 built into the main body 6,which slides in the direction of length of the main body 6.
In this embodiment, supporting means 10 for supporting the pin for forming a hole for inserting an indwelling needle A are provided at the distal end of the sliding body 7. The main body 6 is formed integrally by connecting two parallel opposing side plates 61 at rear ends thereof. The guides 62 are formed on the inner walls of the two side plates 61 respectively. The guides 72 is also formed on the outer walls 71 on the left and right sides which constitute the sliding body 7 to be inserted between the two side plates 61. The guides 62 and the guides 72 engage in the sliding direction so that the sliding body 7 can be slidable toward the front of the main body 6. A finger stop 91 are formed as driving means 9 at the upper part of the sliding body 7, and the sliding body 7 is adapted to be slid by fingers holding the main body 6.

In this first embodiment, since gap formed between the distal end of the main body 6 and the distal end of the sliding body 7 serves as a wing holding means 100 for holding the wings 4 in a state in which the pin for forming a hole for inserting an indwelling needle shown in Fig. 5 is attached to the jig for installing the pin B, the pin for forming a hole for inserting an indwelling needle A before mounting is fixedly attached to the distal end of the jig B without swinging of the two wings 4, and the direction of the hole-forming pin becomes stable. The hole-forming pin can be reliably inserted in the predetermined direction; therefore operability of the jig is good.

### (Example 2 of the jig for installing the pin)

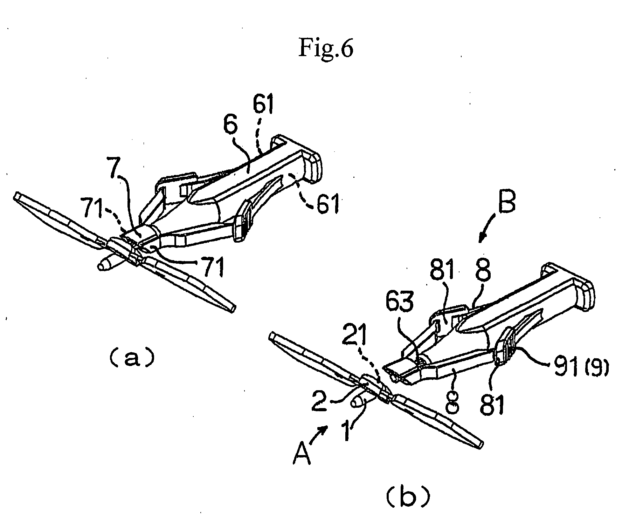

In Fig. 6 showing a second embodiment of the jig B used for the hole-forming pin of the present invention, the sliding body 7 is slidably inserted into a sliding shaft 63 projecting from the distal end of the main body 6. The ends of two expansible arm-shaped spring parts 8 bent in the outward direction of the main body 6 and the sliding body 7 are fixed to the left and right side walls 61 of the main body 6 and the left and right side walls 71 of the sliding body 7, respectively. The spring parts 8 become bendable by using pin connecting parts 81 to cause the sliding movement of the sliding body 7 by the difference of the distance between the both ends of the spring parts 8, which are generated by expansion and contraction of the spring parts 8. Finger stop parts 91 is formed in the pin connecting part 81 of the spring part 8 as the driving means 9, and the sliding body 7 is adapted to slide with respect to the sliding shaft 63 by pushing the left and right finger stop parts 91 with fingertips.

In the second embodiment, the distal end of the sliding shaft 63 projecting from the distal end of the main body 6 is fitted into an insertion hole 21 (not shown in the drawing) formed on the opposite side from the projecting side of the insertion part 1 of the insertion stop part 2 of the pin for forming a hole for inserting an indwelling needle A and works as the supporting means 10 for supporting the pin for forming a hole for inserting an indwelling needle.
The supporting means 10, in other word the distal end of the sliding shaft 63 in this case, is moved relatively backward to the sliding body 7 in association with the above-described sliding movement, whereby the pin for forming a hole for inserting an indwelling needle is separated from the supporting means 10. Accordingly, the jig can be removed immediately after inserting the hole-forming pin from the skin surface toward the blood vessel. Therefore, the operation such as to remove the hole-forming pin from the jig with other medical jigs or the like is not necessary, and hole-forming pin can be fixed to the skin surface immediately with the wings by adhesive tape or the like, thereby achieving good operability.

### (Example 3 of the jig for installing the pin)

In Fig. 7 (Fig. 8) showing a third embodiment of the jig B used in the hole-forming pin according to the present invention, the jig B comprises a main body 6 and the sliding body 7 that is built in the main body 6 and slides in the longitudinal direction of the main body 6, the main body 6 being tubular shape having left and right side walls 65, a bottom wall 66, a top plate 67, and a rear end wall 68. A coil spring 90 is fitted between the inside of the rear end wall 68 of the main body 6 and the rear end 74 of the sliding body 7 so that the sliding body 7 is energized to the front. The sliding body 7 is formed with a forwardly facing inclined surface 73 on upper part thereof, a rearwardly facing inclined surface 93 corresponding to the forwardly facing inclined surface 73 is formed on the lower part of the finger stop part 92, and the upper part of the finger stop part 92 projects upwardly of the main body 6 through a through hole 64 formed on the top plate 67 of the main body 6. Then, when the finger stop part 92 is pressed downward, the rearwardly facing inclined surface 93 presses the forwardly facing inclined surface 73 of the sliding body 7, so that the sliding body 7 is slid rearwardly of the main body 6 in the compression direction of the coil spring 90.

At the front end of the sliding body 7, two wing supporting pins 75 connected to the front end of the sliding body 7 project obliquely forwardly of the main body through the through holes 64' formed at the front end of the main body 6, and the distal ends of the wind supporting pins 75 are fitted into two small holes 41 formed on the wings 4 of the pin for forming a hole for inserting an indwelling needle A. The front end has a function as the supporting means 10 that support the hole-forming pin A and simultaneously, a function as the wing holding means 100 for holding the wings 4. As in the case of the second embodiment described above, the supporting means 10 moves, relatively backward to the main body 6 when the sliding body 7 slides, and the wing supporting pins 75 are retracted from the through holes 64' formed at the distal end of the main body 6 to separate the pin for forming a hole for inserting an indwelling needle A from the supporting means 10 so that the jig B can be separated immediately after having inserted the hole-forming pin A from the skin surface toward the blood vessel.

As the material of the above-described jig B, injection-molded product of thermoplastic synthetic resin can be used for the finger stop part and the pins supporting a wing part, as well as the main body, the sliding body generally, but cast product of alloy, cut product of metal, and the like also can be used. Also, the spring material for the expansible arm-shaped spring may be preferably formed of corrosion-resisting metal material, and the coil spring formed of corrosion-resistant metallic wire material such as stainless steel is used.

### Industrial Applicability

The usage of the pin for forming a hole for inserting an indwelling needle of the present invention will be described first. First, the process of dialysis or the like starts by puncturing the dialysis-indwelling needle having a sharp edge into a vein of a patient. The dialysis-indwelling needle is pulled out after the process of the hemodialysis is completed. Since a passage is formed as a puncture passage immediately after having removed the dialysis-indwelling needle, the hole-forming pin according to the present invention is inserted along the passage.

By removing the hole-forming pin which is indwelt for several days with fixing the wings 4 to the arm with adhesive tape, an indwelling needle insertion hole (button hole) extending from the skin surface to the interior of the blood vessel is formed to the wall of the blood vessel or to the position close to the blood vessel.

Therefore, for the next treatment, by puncturing the wall of the blood vessel with the puncturing needle only once in this point, the passage is pierced from the skin surface to the interior of the blood vessel.

When it is not used, the buttonhole is clogged by blood that is solid-state or semi-solid and slough is formed on the surface of the entrance of the buttonhole.

Therefore, when the dialysis-indwelling needle indwells in the buttonhole, the slough is removed with a medical hook or tweezers so that the indwelling needle can be inserted into the hole. As the dialysis-indwelling needle to be inserted into the buttonhole, the one whose distal end is not sharp is preferably selected, whereby it causes the patient less pain.

As described above, with the pin for forming a hole for inserting an indwelling needle, even when the angle of the puncture passage which appears following the removal of the indwelling needle and the angle of the hole-forming pin are not necessarily identical, the insertion part can be inserted smoothly along the angle of the puncture passage owing to flexibility of the elastic joint parts provided on the pin for forming a hole for inserting an indwelling needle. Since the pin for forming a hole for inserting an indwelling needle does not rotate while it is indwelling, the buttonhole can be formed in an intended direction and angle.

Since the button hole which become an passage to be inserted the indwelling needle can be easily formed by the pin for forming a hole for inserting an indwelling needle in the present invention, during indwelling of the indwelling needle, the burden to the living body can be alleviated. During dialysis by the buttonhole method, the burden to the living body also can be alleviated.

Furthermore, since holding the wings can perform the insertion into the puncture, insertion operation is easy even without using the jig. When the jig is used for mounting the hole-forming pin as required, with the jig for installing the hole-forming pin according to the present invention, the hole-forming pin can be attached reliably to the jig, and the jig can be used while confirming the direction of insertion into the living body.

The jig for installing the pin can be removed immediately after inserting the hole-forming pin from the skin surface toward the blood vessel. Therefore, the operation such as to remove the hole-forming pin from the jig with other medical jigs or the like is not necessary, and hole-forming pin can be fixed to the skin surface immediately with the wings by adhesive tape or the like, thereby achieving good operability.

The hole-forming pin for inserting the dialysis indwelling needle has been described in the description of the embodiment above, the pin for forming a hole for inserting an indwelling needle of the present application is widely applied not only to the dialysis, but also the applications such as regular inspection such as blood sampling or continuous or discontinuous administration of drug solution (such as fluid infusion) in accordance with the intended use depending on the setting of the material or dimensions of the inserting part of the indwelling needle hole-forming pin or of the elastic joint part.

## Claims

1. A hole-forming pin for inserting indwelling needle comprising;
a column-shaped insertion part having a curved surface at the distal end thereof,
an insertion stop part provided at the proximal end of the insertion part, and
wings connected to the insertion stop part :
the insertion stop part and the wings are connected through elastic joint parts; said pin can form an hole for inserting an indwelling needle for holding an indwelling needle from a skin surface to a blood vessel.

2. A hole-forming pin for inserting indwelling needle according to Claim 1, wherein the insertion part is from 0.5 to 3.0 mm in outer diameter and is from 3 to 20 mm in length.

3. A hole-forming pin for inserting indwelling needle according to Claim 1 or 2, wherein elastic deformation at the elastic joint parts is such that the entire wings can be moved in the direction of right angle cross-section which is substantially at a right angle to blood flow direction of the blood vessel to which the column-shaped insertion part is to be inserted with respect to the insertion stop part.

4. A hole-forming pin for inserting indwelling needle according to any one of Claims 1 to 3, wherein the elastic deformation at the elastic joint parts allows the column-shaped insertion part to have flexibility in puncturing angle to the blood vessel with respect to the wings, the column-shaped insertion part is to be inserted through the blood vessel.

5. A hole-forming pin for inserting indwelling needle according to any one of Claims 1 to 3, wherein the elastic deformation at the elastic joint parts allows the column-shaped insertion part of the insertion stop part to have angular flexibility substantially in the rotating direction about the axis of the column-shaped part of the column-shaped insertion part with respect to the wings.

6. A hole-forming pin for inserting indwelling needle according to any one of Claims 1 to 3, wherein the elastic deformation at the elastic joint parts is set based on the material and the shape of the elastic joint parts so that the balance with the elastic deformation for allowing the column-shaped insertion part to have flexibility in the puncturing angle to the blood vessel with respect to the wings according to Claim 4 and the elastic deformation for allowing the column-shaped insertion part at the insertion stop part to have angular flexibility substantially in the rotating direction about the axis of the column-shaped part of the column-shaped insertion part with respect to the wings according to Claim 5 are adjusted.

7. A hole-forming pin for inserting indwelling needle according to Claim 6, wherein the wings are connected to the insertion stop part via flexible joint branches.

8. A hole-forming pin for inserting indwelling needle according to Claim 7, wherein the joint branches are from 0.1 to 2 mm in diameter of the lateral cross-section, and 0.5 to 10 mm in length.

9. A jig for installing a pin for forming a hole for inserting indwelling needle comprising a main body and a sliding body that is built in the main body for sliding in the longitudinal direction of the main body; the supporting means supporting the pin for forming a hole for inserting indwelling needle for holding the indwelling needle from the skin surface toward the blood vessel are provided at the distal end(s) of the main body and/or the sliding body: the jig is for holding a indwelling needle from a skin surface toward a blood vessel

10. A jig for installing a pin for forming a hole for inserting indwelling needle according to Claim 9, wherein the main body is integrally formed with two opposing side plates extending in parallel with each other,
the sliding body is fitted between the two side plates,
a guide engages the inner walls of the two side plates which constitute the main body and the outer wall which constitutes the sliding body, and
the sliding body can slide forwardly of the main body by forming the guide.

11. A jig for installing a pin for forming a hole for inserting indwelling needle according to Claim 9, wherein the sliding body slidably inserted into a sliding shaft projecting from the distal end of the main body,
the two spring parts bending in the outward direction of the main body and the sliding body ,
two spring parts are fixed at the ends thereof to the left and right side walls of the main body and the left and right side walls of the sliding body respectively.

12. A jig for installing a pin for forming a hole for inserting indwelling needle according to any one of Claims 9 to 11, wherein the sliding body is provided with driving means that allows the sliding body to slide along the main body.

13. A jig for installing a pin for forming a hole for inserting indwelling needle according to Claim 12, wherein the driving means used for the sliding body is using a finger stop part formed on a part of the sliding body.

14. A jig for installing a pin for forming a hole for inserting indwelling needle according to Claim 12, wherein the driving means for the sliding body comprises the finger stop part and the spring; the driving means for the sliding body is built in the tubular main body formed of left and right side walls, a bottom plate, a top plate and a rear end wall; a coil spring is fitted between the interior of the rear end wall of the main body and the rear end of the sliding body so that the sliding body is energized forward; an upper part of the sliding body is formed with inclined plane facing a forwardly; a inclined plane facing rearwardly corresponding to said inclined plane is formed on the lower part of the finger stop part , and the upper part of the finger hook part projects above the main body through a through-hole formed on the top plate of the main body.

15. A jig for installing a pin for forming a hole for inserting indwelling needle according to any one of Claims 9 to 14, wherein at least one part of the supporting means for supporting the indwelling needle insertion hole-forming pin is retracted relatively rearward of the distal end of the main body in association with the sliding movement and the indwelling needle insertion hole-forming pin is separated from the supporting means.

16. A jig for installing a pin for forming a hole for inserting indwelling needle according to any one of Claims 9 to 15, comprising wing holding means for holding the wings of the indwelling needle insertion hole-forming pin at the distal end of the main body and/or the distal end of the sliding body.
